# EUROPEAN PATENT SPECIFICATION

(11) **EP 2 917 347 B1**
(45) Date of publication and mention of the grant of the patent: **02.01.2019**
(21) Application number: 13786515.0
(22) Date of filing: 07.11.2013
(51) Int. Cl.: C12N 15/113, C07K 16/28, C07K 16/30, G01N 33/574, A61P 35/04

(54) **METHODS AND PHARMACEUTICAL COMPOSITIONS FOR THE TREATMENT OF BONE METASTASES**
VERFAHREN UND PHARMAZEUTISCHE ZUSAMMENSETZUNGEN ZUR BEHANDLUNG VON KNOCHENMETASTASEN
PROCÉDÉS ET COMPOSITIONS PHARMACEUTIQUES POUR LE TRAITEMENT DES MÉTASTASES OSSEUSES

(30) Priority: 08.11.2012 EP 12306376; 12.03.2013 US 201361777518 P
(43) Date of publication of application: 16.09.2015
(73) Proprietor: Institut National de la Santé et de la Recherche Médicale (INSERM), 75013 Paris (FR); Université Claude Bernard Lyon 1, 69622 Villeurbanne, Cedex (FR)
(72) Inventor: CLEZARDIN, Philippe, F-69372 Lyon cedex 08 (FR); ECKEL, Bénédicte, F-69007 Lyon (FR); DIAZ-LATOUD, Chantal, F-69372 Lyon cedex 08 (FR); CLEMENT-DEMANGE, Lise, F-69372 Lyon cedex 08 (FR)
(74) Representative: Inserm Transfert
(86) International application number: PCT/EP2013/073284
(87) International publication number: WO 2014/072416

(56) References cited:
- WO-A2-02/36771
- WO-A2-2008/100805
- RACHEL THERIAULT ET AL: "Biology of Bone Metastases", CANCER CONTROL, vol. 19, no. 2, 1 April 2012 (2012-04-01), pages 92-101, XP055057708,
- DIEL I J ET AL: "Serum bone sialoprotein in patients with primary breast cancer is a prognostic marker for subsequent bone metastasis", CLINICAL CANCER RESEARCH, vol. 5, no. 12, 1 December 1999 (1999-12-01), pages 3914-3919, XP002230791, THE AMERICAN ASSOCIATION FOR CANCER RESEARCH, US ISSN: 1078-0432
- KAUR SUKHBIR ET AL: "Silencing of directional migration in roundabout4 knockdown endothelial cells", BMC CELL BIOLOGY, vol. 9, no. 1, 3 November 2008 (2008-11-03), page 61, XP021047599, BIOMED CENTRAL, LONDON, GB ISSN: 1471-2121, DOI: 10.1186/1471-2121-9-61
- SUCHTING ET AL: "Soluble Robo4 receptor inhibits in vivo angiogenesis and endothelial cells migration", FASEB JOURNAL, vol. 19, no. 1, 1 October 2005 (2005-10-01), pages 121-123, XP008109831, FED. OF AMERICAN SOC. FOR EXPERIMENTAL BIOLOGY, US ISSN: 0892-6638, DOI: 10.1096/FJ.04-1991FJE
- BALLARD MIMMI S ET AL: "A Roundabout Way to Cancer", ADVANCES IN CANCER RESEARCH; GUIDANCE MOLECULES IN CANCER AND TUMOR ANGIOGENESIS, vol. 114, 1 January 2012 (2012-01-01), pages 187-235, XP008160909, ACADEMIC PRESS, US ISSN: 0065-230X, DOI: 10.1016/B978-0-12-386503-8.00005-3 [retrieved on 2012-05-12]
- GÖRN M ET AL: "Serum levels of Magic Roundabout protein in patients with advanced non-small cell lung cancer (NSCLC)", LUNG CANCER, vol. 49, no. 1, 1 July 2005 (2005-07-01), pages 71-76, XP027774231, ELSEVIER, AMSTERDAM, NL ISSN: 0169-5002 [retrieved on 2005-07-01]
- BÉNÉDICTE ECKEL ET AL: "S16: Involvement of the Robo 1 and 4 Proteins in Breast Cancer Bone Metastasis", IBMS BONEKEY | 12TH INTERNATIONAL CONFERENCE ON CANCER-INDUCED BONE DISEASE, vol. 9, 193, 14 November 2012 (2012-11-14) , pages S1, S6-S7, XP002694511, DOI: 10.1038/bonekey.2012.193

### SEQUENCE LISTING

<110> INSERM
<120> METHODS AND PHARMACEUTICAL COMPOSITIONS FOR THE TREATMENT OF BONE METASTASES
<130> BIO12095 CLEZARDIN / MC
<160> 4
<170> PatentIn version 3.3
<210> 1
   <211> 56
   <212> DNA
   <213> Artificial
<220>
   <223> ROBO1 ShRNA forward
<400> 1
   accgcagtac taagggaaca ataagttctc tattgttccc ttagtactgc tttttc 56
<210> 2
   <211> 56
   <212> DNA
   <213> Artificial
<220>
   <223> ROBO1 ShRNA reverse
<400> 2
   tgcagaaaaa gcagtactaa gggaacaata gagaacttat tgttccctta gtactg 56
<210> 3
   <211> 19
   <212> DNA
   <213> Artificial
<220>
   <223> ROBO4 ShRNA forward
<400> 3
   gagcagagaa gagtgacga 19
<210> 4
   <211> 19
   <212> DNA
   <213> Artificial
<220>
   <223> ROBO4 ShRNA reverse
<400> 4
   tcttacacgg ccttgttca 19

## Description

### FIELD:

The present invention relates to methods and pharmaceutical compositions for the treatment of bone metastases.

### BACKGROUND:

Bone metastases, the spread of cancer to the bones, occur in more than 1.5 million patients with cancer world-wide and are most commonly associated with cancers of the prostate, lung, and breast (Weilbaecher et al., Nat Rev Cancer 2011; 11:411-24). Metastatic cancer cells residing in the bone marrow alter the functions of bone-resorbing (osteoclasts) and bone-forming (osteoblasts) cells and hijack signals coming from the bone matrix (Weilbaecher et al., Nat Rev Cancer 2011; 11:411-24). By disrupting the physiological balance between bone resorption and bone formation, metastatic cells therefore promote skeletal destruction. The realization that metastatic cells in the bone marrow alter the functions of osteoclasts and osteoblasts has led to the use of therapies targeting osteoclasts (bisphosphonates, denosumab). Many therapeutic agents for the treatment and prevention of bone metastases was described in the prior art (Rachel Theriault et al. "Biology of Bane Metastases", Cancer Control, vol. 19, no. 2, 1 April 2012 (2012-04-01)). For instance, bisphosphonates inhibit the activity of bone-resorbing osteoclasts, and are the standards of care for delaying bone destruction in cancer patients with bone metastases (Gnant & Clezardin, Cancer Treat Rev 2011; Brown & Coleman, Nat Rev Clin Oncol 2012; 9:110-8). Denosumab, a fully humanized monoclonal antibody that specifically binds to the receptor activator of NF-κB ligand (RANKL), inhibits the formation of osteoclasts, thereby reducing bone destruction (Brown & Coleman, Nat Rev Clin Oncol 2012; 9:110-8). These treatments however are only palliative and are often associated with adverse side effects. Thus, it is vital to better understand molecular mechanisms that precede the overt development of skeletal lesions in order to develop drugs that target cancer cells early during metastasis formation, when they colonize the bone marrow.

It was reported that bone sialoprotein in serum of primary breast cancer patients is a prognostic marker for subsequent bone metastasis (Diel I. et al. "Serum bone sialoprotein in patients with primary breast cancer is a prognostic marker for subsequent bane metastasis", Clinical Cancer Research, The American Association For Cancer Research, US, vol. 5, no. 12, 1 December 1999 (1999-12-01)).

It was reported that ROBO4 siRNA abrogates serum-mediated migration of endothelial cells (Kaur Sukhbir et al. "Silencing of directional migration in roundabout 4 knockdown endothelial cells", BMC Cell Biology, Biomed Central, London, GB, vol. 9, no. 1, 3 November 2008 (2008-11-03)). Soluble ROBO4 receptor Robo4Fc was shown to inhibit *in vivo* angiogenesis and endothelial cell migration (Suchting et al: "Soluble Robo4 receptor inhibits in vivo angiogenesis and endothelial cells migration", FASEB Journal, Fed. Of American Soc. for Experimental Biology, US, vol. 19, no. 1, 1 October 2005 (2005-10-01)). WO2008/100805 discloses than an anti-ROBO4 antibody inhibits HUVEC tube elongation but also teaches that naked anti-ROB04 antibody has no anti-cancer effect in vivo.

### SUMMARY:

The present invention relates to methods and pharmaceutical compositions for the treatment of bone metastases. In particular, the present invention is defined by the claims.

More particularly the first object of the present invention relates to an agent selected from the group consisting of an anti-ROBO4 antibody, a ROBO4 decoy polypeptide, and an inhibitor of ROB04 expression for use in a method for preventing or treating bone metastases in a subject in need thereof wherein the inhibitor of ROBO4 expression is selected from the group consisting of ROBO4 siRNAs, ROBO4 shRNAs, ROBO4 ribozymes and ROBO4 antisense oligonucleotides and wherein the ROBO4 decoy polypeptide comprises the extracellular domain of ROB04.

A second object of the present invention relates to a method of testing whether a patient with a cancer is at risk of having bone metastases comprising i) determining the expression level of ROBO4 in a sample obtained from said patient ii) comparing the level determined at step i) with a predetermined reference level and iii) concluding that the patient has a high risk of having bone metastases when the level determined at step i) is higher than the predetermined reference level.

### DETAILED DESCRIPTION:

The inventors surmise that ROBO receptors support disseminated tumor cells (DTC) functions, allowing these cells to adapt and thrive in the bone marrow. Using human B02 breast cancer cells, a subpopulation of the MDA-MB-231 cell line which metastasizes to bone only, a gene expression microarray analysis showed that ROBO1 and ROBO4 were significantly overexpressed in B02 cells compare to that observed with parental cells. ROBO2 and ROBO3 were not expressed in both cell lines. The functional role of ROBO1/ROBO4 in B02 breast cancer metastasis was therefore assessed, using shRNAs targeting each of these receptors. Consistent with its role as a tumor suppressor gene, ROBO1 silencing enhanced migratory and invasive properties of shROBO1 clones in vitro, and it increased growth of shROBO1 tumor xenografts in animals. Moreover, ROBO1 silencing promoted bone marrow micrometastasis formation ex vivo. In sharp contrast, ROBO4 silencing inhibited shROBO4 breast cancer cell migration and invasion in vitro, decreased orthotopic tumor growth in vivo and reduced ex-vivo formation of bone marrow micrometastases. Thus, the data uncovered a previously unknown function of ROBO receptors in bone marrow micrometastasis formation and revealed that ROBO1 and ROBO4 have opposite functions.

Accordingly the present invention relates to an agent selected from the group consisting of an anti-ROBO4 antibody, a ROBO4 decoy polypeptide, an inhibitor of ROBO4 expression for use in a method for preventing or treating bone metastases in a subject in need thereof wherein the inhibitor of ROBO4 expression is selected from the group consisting of ROBO4 siRNAs, ROBO4 shRNAs, ROBO4 ribozymes and ROBO4 antisense oligonucleotides and wherein the ROBO4 decoy polypeptide comprises the extracellular domain of ROBO4.

The terms "subject," and "patient," used interchangeably herein, refer to a mammal, particularly a human suffering from a cancer such as prostate cancer, breast cancer, lung cancer, melanoma, pancreatic cancer, colorectal cancer, ovarian cancer and brain cancer.

As used herein the term "ROBO4" has its general meaning in the art and denotes the cell surface transmembrane protein Roundabout 4. ROBO4 was first described as an axon guidance receptor protein, and its amino acid sequence and the gene sequence coding thereof are disclosed in GenBank ID [acc. no. AF361473].

As used herein, "antibody" includes both naturally occurring and non-naturally occurring antibodies. Specifically, "antibody" includes polyclonal and monoclonal antibodies, and monovalent and divalent fragments thereof. Furthermore, "antibody" includes chimeric antibodies, wholly synthetic antibodies, single chain antibodies, and fragments thereof. The antibody may be a human or non human antibody. A non human antibody may be humanized by recombinant methods to reduce its immunogenicity in man.

Antibodies may be prepared according to conventional methodology. Monoclonal antibodies may be generated using the method of Kohler and Milstein (Nature, 256:495, 1975). To prepare monoclonal antibodies useful in the invention, a mouse or other appropriate host animal is immunized at suitable intervals (e.g., twice-weekly, weekly, twice-monthly or monthly) with antigenic forms of ROB04. The animal may be administered a final "boost" of antigen within one week of sacrifice. It is often desirable to use an immunologic adjuvant during immunization. Suitable immunologic adjuvants include Freund's complete adjuvant, Freund's incomplete adjuvant, alum, Ribi adjuvant, Hunter's Titermax, saponin adjuvants such as QS21 or Quil A, or CpG-containing immunostimulatory oligonucleotides. Other suitable adjuvants are well-known in the field. The animals may be immunized by subcutaneous, intraperitoneal, intramuscular, intravenous, intranasal or other routes. A given animal may be immunized with multiple forms of the antigen by multiple routes.

Briefly, recombinant forms of ROBO4 may be provided using any previously described method. Following the immunization regimen, lymphocytes are isolated from the spleen, lymph node or other organ of the animal and fused with a suitable myeloma cell line using an agent such as polyethylene glycol to form a hydridoma. Following fusion, cells are placed in media permissive for growth of hybridomas but not the fusion partners using standard methods, as described (Coding, Monoclonal Antibodies: Principles and Practice: Production and Application of Monoclonal Antibodies in Cell Biology, Biochemistry and Immunology, 3rd edition, Academic Press, New York, 1996). Following culture of the hybridomas, cell supernatants are analyzed for the presence of antibodies of the desired specificity, i.e., that selectively bind the antigen. Suitable analytical techniques include ELISA, flow cytometry, immunoprecipitation, and western blotting. Other screening techniques are well-known in the field. Preferred techniques are those that confirm binding of antibodies to conformationally intact, natively folded antigen, such as non-denaturing ELISA, flow cytometry, and immunoprecipitation.

Significantly, as is well-known in the art, only a small portion of an antibody molecule, the paratope, is involved in the binding of the antibody to its epitope. The Fc' and Fc regions, for example, are effectors of the complement cascade but are not involved in antigen binding. An antibody from which the pFc' region has been enzymatically cleaved, or which has been produced without the pFc' region, designated an F(ab')₂ fragment, retains both of the antigen binding sites of an intact antibody. Similarly, an antibody from which the Fc region has been enzymatically cleaved, or which has been produced without the Fc region, designated a Fab fragment, retains one of the antigen binding sites of an intact antibody molecule. Proceeding further, Fab fragments consist of a covalently bound antibody light chain and a portion of the antibody heavy chain denoted Fd. The Fd fragments are the major determinant of antibody specificity (a single Fd fragment may be associated with up to ten different light chains without altering antibody specificity) and Fd fragments retain epitope-binding ability in isolation.

Within the antigen-binding portion of an antibody, as is well-known in the art, there are complementarity determining regions (CDRs), which directly interact with the epitope of the antigen, and framework regions (FRs), which maintain the tertiary structure of the paratope. In both the heavy chain Fd fragment and the light chain of IgG immunoglobulins, there are four framework regions (FR1 through FR4) separated respectively by three complementarity determining regions (CDR1 through CDRS). The CDRs, and in particular the CDRS regions, and more particularly the heavy chain CDRS, are largely responsible for antibody specificity.

It is now well-established in the art that the non CDR regions of a mammalian antibody may be replaced with similar regions of conspecific or heterospecific antibodies while retaining the epitopic specificity of the original antibody. This is most clearly manifested in the development and use of "humanized" antibodies in which non-human CDRs are covalently joined to human FR and/or Fc/pFc' regions to produce a functional antibody.

This disclosure provides in certain embodiments compositions and methods that include humanized forms of antibodies. As used herein, "humanized" describes antibodies wherein some, most or all of the amino acids outside the CDR regions are replaced with corresponding amino acids derived from human immunoglobulin molecules. Methods of humanization include, but are not limited to, those described in U.S. Pat. Nos. 4,816,567,5,225,539,5,585,089, 5,693,761, 5,693,762 and 5,859,205. The above U.S. Pat. Nos. 5,585,089 and 5,693,761, and WO 90/07861 also propose four possible criteria which may used in designing the humanized antibodies. The first proposal was that for an acceptor, use a framework from a particular human immunoglobulin that is unusually homologous to the donor immunoglobulin to be humanized, or use a consensus framework from many human antibodies. The second proposal was that if an amino acid in the framework of the human immunoglobulin is unusual and the donor amino acid at that position is typical for human sequences, then the donor amino acid rather than the acceptor may be selected. The third proposal was that in the positions immediately adjacent to the 3 CDRs in the humanized immunoglobulin chain, the donor amino acid rather than the acceptor amino acid may be selected. The fourth proposal was to use the donor amino acid reside at the framework positions at which the amino acid is predicted to have a side chain atom within 3A of the CDRs in a three dimensional model of the antibody and is predicted to be capable of interacting with the CDRs. The above methods are merely illustrative of some of the methods that one skilled in the art could employ to make humanized antibodies. One of ordinary skill in the art will be familiar with other methods for antibody humanization.

In one embodiment of the humanized forms of the antibodies, some, most or all of the amino acids outside the CDR regions have been replaced with amino acids from human immunoglobulin molecules but where some, most or all amino acids within one or more CDR regions are unchanged. Small additions, deletions, insertions, substitutions or modifications of amino acids are permissible as long as they would not abrogate the ability of the antibody to bind a given antigen. Suitable human immunoglobulin molecules would include IgG1, IgG2, IgG3, IgG4, IgA and IgM molecules. A "humanized" antibody retains a similar antigenic specificity as the original antibody. However, using certain methods of humanization, the affinity and/or specificity of binding of the antibody may be increased using methods of "directed evolution", as described by Wu et al., /. Mol. Biol. 294:151, 1999.

Fully human monoclonal antibodies also can be prepared by immunizing mice transgenic for large portions of human immunoglobulin heavy and light chain loci. See, e.g., U.S. Pat. Nos. 5,591,669, 5,598,369, 5,545,806, 5,545,807, 6,150,584, and references cited therein. These animals have been genetically modified such that there is a functional deletion in the production of endogenous (e.g., murine) antibodies. The animals are further modified to contain all or a portion of the human germ-line immunoglobulin gene locus such that immunization of these animals will result in the production of fully human antibodies to the antigen of interest. Following immunization of these mice (e.g., XenoMouse (Abgenix), HuMAb mice (Medarex/GenPharm)), monoclonal antibodies can be prepared according to standard hybridoma technology. These monoclonal antibodies will have human immunoglobulin amino acid sequences and therefore will not provoke human anti-mouse antibody (KAMA) responses when administered to humans.

In vitro methods also exist for producing human antibodies. These include phage display technology (U.S. Pat. Nos. 5,565,332 and 5,573,905) and in vitro stimulation of human B cells (U.S. Pat. Nos. 5,229,275 and 5,567,610).

Thus, as will be apparent to one of ordinary skill in the art, the present disclosure also provides for F(ab') 2 Fab, Fv and Fd fragments; chimeric antibodies in which the Fc and/or FR and/or CDR1 and/or CDR2 and/or light chain CDR3 regions have been replaced by homologous human or non-human sequences; chimeric F(ab')₂ fragment antibodies in which the FR and/or CDR1 and/or CDR2 and/or light chain CDR3 regions have been replaced by homologous human or non-human sequences; chimeric Fab fragment antibodies in which the FR and/or CDR1 and/or CDR2 and/or light chain CDR3 regions have been replaced by homologous human or non-human sequences; and chimeric Fd fragment antibodies in which the FR and/or CDR1 and/or CDR2 regions have been replaced by homologous human or non-human sequences. The present disclosure also includes so-called single chain antibodies.

The various antibody molecules and fragments may derive from any of the commonly known immunoglobulin classes, including but not limited to IgA, secretory IgA, IgE, IgG and IgM. IgG subclasses are also well known to those in the art and include but are not limited to human IgG1, IgG2, IgG3 and IgG4.

In another embodiment, the antibody is a single domain antibody. The term "single domain antibody" (sdAb) or "VHH" refers to the single heavy chain variable domain of antibodies of the type that can be found in Camelid mammals which are naturally devoid of light chains. Such VHH are also called "nanobody®". According to the disclosure, sdAb can particularly be llama sdAb.

As used herein the term "aptamer" refers to a a class of molecule that represents an alternative to antibodies in term of molecular recognition. Aptamers are oligonucleotide or oligopeptide sequences with the capacity to recognize virtually any class of target molecules with high affinity and specificity. Accordingly the term "anti-ROBO4 aptamer" refers to an apatamer directed against ROBO4. Aptamers may be isolated through Systematic Evolution of Ligands by EXponential enrichment (SELEX) of a random sequence library. The random sequence library is obtainable by combinatorial chemical synthesis of DNA.

In one embodiment the agent is a ROBO4 decoy polypeptide that is capable of trapping the ligands of ROBO4 and thus preventing its activation. Typically, the ROBO4 decoy polypeptide comprises the extracellular domain of ROBO4 and therefore includes soluble forms of ROB04. A suitable soluble form of ROB04 might comprise, for example, a truncated form of the protein from which the transmembrane domain has been removed by chemical, proteolytic or recombinant methods.

In a particular embodiment, ROBO4 decoy polypeptide comprises the extracellular domain of ROBO4 fused to the Fc domain of an immunoglobulin. The Fc domain of said fusion protein serves at least one of the following purposes: secretion of the fusion protein from cells that produce said fusion protein, providing the extracellular domain of ROB04 in a form (e.g. folding or aggregation state) functional for trapping the ligands of ROBO4, affinity purification of said fusion protein, recognition of the fusion protein by an antibody, providing favourable properties to the fusion protein when used as a medicament. Suitable immunoglobins are IgG, IgM, IgA, IgD, and IgE. IgG and IgA are preferred IgGs are most preferred, e.g. an IgG1. Said Fc domain may be a complete Fc domain or a function-conservative variant thereof. A variant of Fc is function-conservative if it retains at least one of the functions listed above. The domains of the fusion protein may be linked by a linker. The linker may consist of about 1 to 100, preferably 1 to 10 amino acid residues.

The polypeptides may be produced by any suitable means, as will be apparent to those of skill in the art. In order to produce sufficient amounts of the polypeptide for use in accordance with the present disclosure, expression may conveniently be achieved by culturing under appropriate conditions recombinant host cells containing the polypeptide of the disclosure. Preferably, the polypeptide is produced by recombinant means, by expression from an encoding nucleic acid molecule. Systems for cloning and expression of a polypeptide in a variety of different host cells are well known. When expressed in recombinant form, the polypeptide is preferably generated by expression from an encoding nucleic acid in a host cell. Any host cell may be used, depending upon the individual requirements of a particular system. Suitable host cells include bacteria mammalian cells, plant cells, yeast and baculovirus systems. Mammalian cell lines available in the art for expression of a heterologous polypeptide include Chinese hamster ovary cells. HeLa cells, baby hamster kidney cells and many others. Bacteria are also preferred hosts for the production of recombinant protein, due to the ease with which bacteria may be manipulated and grown. A common, preferred bacterial host is E coli.

In specific embodiments, it is contemplated that polypeptides used in the therapeutic methods of the present disclosure may be modified in order to improve their therapeutic efficacy. Such modification of therapeutic compounds may be used to decrease toxicity, increase circulatory time, or modify biodistribution. For example, the toxicity of potentially important therapeutic compounds can be decreased significantly by combination with a variety of drug carrier vehicles that modify biodistribution. In example adding dipeptides can improve the penetration of a circulating agent in the eye through the blood retinal barrier by using endogenous transporters.

A strategy for improving drug viability is the utilization of water-soluble polymers. Various water-soluble polymers have been shown to modify biodistribution, improve the mode of cellular uptake, change the permeability through physiological barriers; and modify the rate of clearance from the body. To achieve either a targeting or sustained-release effect, water-soluble polymers have been synthesized that contain drug moieties as terminal groups, as part of the backbone, or as pendent groups on the polymer chain.

Polyethylene glycol (PEG) has been widely used as a drug carrier, given its high degree of biocompatibility and ease of modification. Attachment to various drugs, proteins, and liposomes has been shown to improve residence time and decrease toxicity. PEG can be coupled to active agents through the hydroxyl groups at the ends of the chain and via other chemical methods; however, PEG itself is limited to at most two active agents per molecule. In a different approach, copolymers of PEG and amino acids were explored as novel biomaterials which would retain the biocompatibility properties of PEG, but which would have the added advantage of numerous attachment points per molecule (providing greater drug loading), and which could be synthetically designed to suit a variety of applications.

Those of skill in the art are aware of PEGylation techniques for the effective modification of drugs. For example, drug delivery polymers that consist of alternating polymers of PEG and tri-functional monomers such as lysine have been used by VectraMed (Plainsboro, N.J.). The PEG chains (typically 2000 daltons or less) are linked to the a- and e-amino groups of lysine through stable urethane linkages. Such copolymers retain the desirable properties of PEG, while providing reactive pendent groups (the carboxylic acid groups of lysine) at strictly controlled and predetermined intervals along the polymer chain. The reactive pendent groups can be used for derivatization, cross-linking, or conjugation with other molecules. These polymers are useful in producing stable, long-circulating pro-drugs by varying the molecular weight of the polymer, the molecular weight of the PEG segments, and the cleavable linkage between the drug and the polymer. The molecular weight of the PEG segments affects the spacing of the drug/linking group complex and the amount of drug per molecular weight of conjugate (smaller PEG segments provides greater drug loading). In general, increasing the overall molecular weight of the block co-polymer conjugate will increase the circulatory half-life of the conjugate. Nevertheless, the conjugate must either be readily degradable or have a molecular weight below the threshold-limiting glomular filtration (e.g., less than 45 kDa).

In addition, to the polymer backbone being important in maintaining circulatory half-life, and biodistribution, linkers may be used to maintain the therapeutic agent in a pro-drug form until released from the backbone polymer by a specific trigger, typically enzyme activity in the targeted tissue. For example, this type of tissue activated drug delivery is particularly useful where delivery to a specific site of biodistribution is required and the therapeutic agent is released at or near the site of pathology. Linking group libraries for use in activated drug delivery are known to those of skill in the art and may be based on enzyme kinetics, prevalence of active enzyme, and cleavage specificity of the selected disease-specific enzymes. Such linkers may be used in modifying the protein or fragment of the protein described herein for therapeutic delivery.

An "inhibitor of ROBO4 expression" refers to a natural or synthetic compound that has a biological effect to inhibit or significantly reduce the expression of ROBO4 gene.

Inhibitors of expression for use in the present disclosure may be based on anti-sense oligonucleotide constructs. Anti-sense oligonucleotides, including anti-sense RNA molecules and anti-sense DNA molecules, would act to directly block the translation of ROB04 mRNA by binding thereto and thus preventing protein translation or increasing mRNA degradation, thus decreasing the level of ROBO4, and thus activity, in a cell. For example, antisense oligonucleotides of at least about 15 bases and complementary to unique regions of the mRNA transcript sequence encoding ROBO4 can be synthesized, e.g., by conventional phosphodiester techniques and administered by e.g., intravenous injection or infusion. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732).

Small inhibitory RNAs (siRNAs) can also function as inhibitors of expression for use in the present disclosure. ROBO4 gene expression can be reduced by contacting a subject or cell with a small double stranded RNA (dsRNA), or a vector or construct causing the production of a small double stranded RNA, such that ROBO4 gene expression is specifically inhibited (i.e. RNA interference or RNAi). Methods for selecting an appropriate dsRNA or dsRNA-encoding vector are well known in the art for genes whose sequence is known (e.g. see Tuschl, T. et al. (1999); Elbashir, S. M. et al. (2001); Hannon, GJ. (2002); McManus, MT. et al. (2002); Brummelkamp, TR. et al. (2002); U.S. Pat. Nos. 6,573,099 and 6,506,559; and International Patent Publication Nos. WO 01/36646, WO 99/32619, and WO 01/68836). All or part of the phosphodiester bonds of the siRNAs of the disclosure are advantageously protected. This protection is generally implemented via the chemical route using methods that are known by art. The phosphodiester bonds can be protected, for example, by a thiol or amine functional group or by a phenyl group. The 5'- and/or 3'- ends of the siRNAs of the disclosure are also advantageously protected, for example, using the technique described above for protecting the phosphodiester bonds. The siRNAs sequences advantageously comprise at least twelve contiguous dinucleotides or their derivatives.

As used herein, the term "siRNA derivatives" with respect to the present nucleic acid sequences refers to a nucleic acid having a percentage of identity of at least 90% with erythropoietin or fragment thereof, preferably of at least 95%, as an example of at least 98%, and more preferably of at least 98%.

As used herein, "percentage of identity" between two nucleic acid sequences, means the percentage of identical nucleic acid, between the two sequences to be compared, obtained with the best alignment of said sequences, this percentage being purely statistical and the differences between these two sequences being randomly spread over the nucleic acid acids sequences. As used herein, "best alignment" or "optimal alignment", means the alignment for which the determined percentage of identity (see below) is the highest. Sequences comparison between two nucleic acids sequences are usually realized by comparing these sequences that have been previously align according to the best alignment; this comparison is realized on segments of comparison in order to identify and compared the local regions of similarity. The best sequences alignment to perform comparison can be realized, beside by a manual way, by using the global homology algorithm developed by SMITH and WATERMAN (Ad. App. Math., vol.2, p:482, 1981), by using the local homology algorithm developped by NEDDLEMAN and WUNSCH (J. Mol. Biol., vol.48, p:443, 1970), by using the method of similarities developed by PEARSON and LIPMAN (Proc. Natl. Acd. Sci. USA, vol.85, p:2444, 1988), by using computer softwares using such algorithms (GAP, BESTFIT, BLAST P, BLAST N, FASTA, TFASTA in the Wisconsin Genetics software Package, Genetics Computer Group, 575 Science Dr., Madison, WI USA), by using the MUSCLE multiple alignment algorithms (Edgar, Robert C., Nucleic Acids Research, vol. 32, p: 1792, 2004). To get the best local alignment, one can preferably used BLAST software. The identity percentage between two sequences of nucleic acids is determined by comparing these two sequences optimally aligned, the nucleic acids sequences being able to comprise additions or deletions in respect to the reference sequence in order to get the optimal alignment between these two sequences. The percentage of identity is calculated by determining the number of identical position between these two sequences, and dividing this number by the total number of compared positions, and by multiplying the result obtained by 100 to get the percentage of identity between these two sequences.

shRNAs (short hairpin RNA) can also function as inhibitors of expression for use in the present disclosure.

Ribozymes can also function as inhibitors of expression for use in the present disclosure. Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyze endonucleolytic cleavage of ROBO4 mRNA sequences are thereby useful within the scope of the present disclosure. Specific ribozyme cleavage sites within any potential RNA target are initially identified by scanning the target molecule for ribozyme cleavage sites, which typically include the following sequences, GUA, GUU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable.

Both antisense oligonucleotides and ribozymes useful as inhibitors of expression can be prepared by known methods. These include techniques for chemical synthesis such as, e.g., by solid phase phosphoramadite chemical synthesis. Alternatively, anti-sense RNA molecules can be generated by in vitro or in vivo transcription of DNA sequences encoding the RNA molecule. Such DNA sequences can be incorporated into a wide variety of vectors that incorporate suitable RNA polymerase promoters such as the T7 or SP6 polymerase promoters. Various modifications to the oligonucleotides of the disclosure can be introduced as a means of increasing intracellular stability and half-life. Possible modifications include but are not limited to the addition of flanking sequences of ribonucleotides or desoxyribonucleotides to the 5' and/or 3' ends of the molecule, or the use of phosphorothioate or 2'-O-methyl rather than phosphodiesterase linkages within the oligonucleotide backbone.

Antisense oligonucleotides, siRNAs, shRNAs and ribozymes of the disclosure may be delivered in vivo alone or in association with a vector. In its broadest sense, a "vector" is any vehicle capable of facilitating the transfer of the antisense oligonucleotide, siRNA, shRNA or ribozyme nucleic acid to the cells and preferably cells expressing ROBO4. Preferably, the vector transports the nucleic acid to cells with reduced degradation relative to the extent of degradation that would result in the absence of the vector. In general, the vectors useful in the disclosure include, but are not limited to, plasmids, phagemids, viruses, other vehicles derived from viral or bacterial sources that have been manipulated by the insertion or incorporation of the antisense oligonucleotide, siRNA, shRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rous sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Preferred viral vectors are based on non-cytopathic eukaryotic viruses in which non-essential genes have been replaced with the gene of interest. Non-cytopathic viruses include retroviruses (e.g., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Kriegler, 1990 and in Murry, 1991).

Preferred viruses for certain applications are the adenoviruses and adeno-associated (AAV) viruses, which are double-stranded DNA viruses that have already been approved for human use in gene therapy. Actually 12 different AAV serotypes (AAV1 to 12) are known, each with different tissue tropisms (Wu, Z Mol Ther 2006; 14:316-27). Recombinants AAV are derived from the dependent parvovirus AAV2 (Choi, VW J Virol 2005; 79:6801-07). The adeno-associated virus type 1 to 12 can be engineered to be replication deficient and is capable of infecting a wide range of cell types and species (Wu, Z Mol Ther 2006; 14:316-27). It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well known to those of skill in the art. See e.g. Sambrook et al., 1989. In the last few years, plasmid vectors have been used as DNA vaccines for delivering antigen-encoding genes to cells in vivo. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUC19, pRC/CMV, SV40, and pBlueScript. Other plasmids are well known to those of ordinary skill in the art. Additionally, plasmids may be custom designed using restriction enzymes and ligation reactions to remove and add specific fragments of DNA. Plasmids may be delivered by a variety of routes. For example, the DNA plasmid can be injected by intramuscular, intradermal, subcutaneous, or other routes. The plasmids may be given in an aqueous solution, dried onto gold particles or in association with another DNA delivery system including but not limited to liposomes, dendrimers, cochleate and microencapsulation.

In a preferred embodiment, the antisense oligonucleotide, siRNA, shRNA or ribozyme nucleic acid sequence is under the control of a heterologous regulatory region, e.g., a heterologous promoter.

The agent of the disclosure may be administered in the form of a pharmaceutical composition, as defined below. Preferably, said agent in a therapeutically effective amount. By a "therapeutically effective amount" is meant a sufficient amount of the agent to treat the disease at a reasonable benefit/risk ratio applicable to any medical treatment. It will be understood that the total daily usage of the agent or pharmaceutical composition comprising thereof will be decided by the attending physician within the scope of sound medical judgment. The specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific agent employed; the duration of the treatment; drugs used in combination or coincidental with the specific agent employed; and like factors well known in the medical arts. For example, it is well within the skill of the art to start doses of the agent at levels lower than those required to achieve the desired therapeutic effect and to gradually increase the dosage until the desired effect is achieved. However, the daily dosage of the agent may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the agent for the symptomatic adjustment of the dosage to the subject to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

In a particular embodiment, the agent of the present disclosure may be used in combination with bisphosphonates and/or with an anti-RANKL antibody (e.g. denosumab).

The agent of the disclosure can thus be formulated into pharmaceutical compositions that further comprise a pharmaceutically acceptable carrier, diluent, adjuvant or vehicle. In one embodiment, the present disclosure relates to a pharmaceutical composition comprising an agent of the disclosure described above, and a pharmaceutically acceptable carrier, diluent, adjuvant or vehicle. In one embodiment, the pharmaceutical composition comprises an effective amount of an agent of the present disclosure or a pharmaceutically acceptable salt thereof and a pharmaceutically acceptable carrier, diluent, adjuvant or vehicle. Pharmaceutically acceptable carriers include, for example, pharmaceutical diluents, excipients or carriers suitably selected with respect to the intended form of administration, and consistent with conventional pharmaceutical practices.

A pharmaceutically acceptable carrier may contain inert ingredients which do not unduly inhibit the biological activity of the agent. The pharmaceutically acceptable carriers should be biocompatible, e.g., non-toxic, non-inflammatory, non-immunogenic or devoid of other undesired reactions or side-effects upon the administration to a subject. Standard pharmaceutical formulation techniques can be employed.

The pharmaceutically acceptable carrier, adjuvant, or vehicle, as used herein, includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Remington's Pharmaceutical Sciences, Sixteenth Edition, E. W. Martin (Mack Publishing Co., Easton, Pa., 1980) discloses various carriers used in formulating pharmaceutically acceptable compositions and known techniques for the preparation thereof. Except insofar as any conventional carrier medium is incompatible with the compounds described herein, such as by producing any undesirable biological effect or otherwise interacting in a deleterious manner with any other component(s) of the pharmaceutically acceptable composition, its use is contemplated to be within the scope of this disclosure.

Some examples of materials which can serve as pharmaceutically acceptable carriers include, but are not limited to, ion exchangers, alumina, aluminum stearate, lecithin, serum proteins (such as human serum albumin), buffer substances (such as twin 80, phosphates, glycine, sorbic acid, or potassium sorbate), partial glyceride mixtures of saturated vegetable fatty acids, water, salts or electrolytes (such as protamine sulfate, disodium hydrogen phosphate, potassium hydrogen phosphate, sodium chloride, or zinc salts), colloidal silica, magnesium trisilicate, polyvinyl pyrrolidone, polyacrylates, waxes, polyethylene-polyoxypropylene-block polymers, methylcellulose, hydroxypropyl methylcellulose, wool fat, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol or polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

The compositions described herein may be administered orally, parenterally, by inhalation spray, topically, rectally, nasally, buccally, vaginally or via an implanted reservoir depending on the severity of the disease being treated. The term "parenteral" as used herein includes, but is not limited to, subcutaneous, intravenous, intramuscular, intra-articular, intra-synovial, intrasternal, intrathecal, intrahepatic, intralesional and intracranial injection or infusion techniques.

Sterile injectable forms of the compositions described herein may be aqueous or oleaginous suspension. These suspensions may be formulated according to techniques known in the art using suitable dispersing or wetting agents and suspending agents. The sterile injectable preparation may also be a sterile injectable solution or suspension in a nontoxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

The pharmaceutical compositions described herein may be orally administered in any orally acceptable dosage form including, but not limited to, capsules, tablets, aqueous suspensions or solutions. In the case of tablets for oral use, carriers commonly used include, but are not limited to, lactose and corn starch. Lubricating agents, such as magnesium stearate, are also typically added. For oral administration in a capsule form, useful diluents include lactose and dried cornstarch. When aqueous suspensions are required for oral use, the agent is combined with emulsifying and suspending agents. If desired, certain sweetening, flavoring or coloring agents may also be added.

Alternatively, the pharmaceutical compositions described herein may be administered in the form of suppositories for rectal administration. These can be prepared by mixing the agent with a suitable non-irritating excipient which is solid at room temperature but liquid at rectal temperature and therefore will melt in the rectum to release the drug. Such materials include, but are not limited to, cocoa butter, beeswax and polyethylene glycols.

A further aspect of the invention relates to a method of testing whether a patient with a cancer is at risk of having bone metastases comprising i) determining the expression level of ROBO4 in a sample obtained from said patient ii) comparing the level determined at step i) with a predetermined reference level and iii) concluding that the patient has a high risk of having bone metastases when the level determined at step i) is higher than the predetermined reference level.

As used herein, the term "sample" refers to sample isolated from the primary tumor of the patient or may consist of a collection of circulating tumor cells isolated from the blood sample obtained from the patient.

Determining the expression level of ROBO4 may be assessed by any of a wide variety of well-known methods.

For example, the expression of the gene encoding for ROBO4 is assessed by analyzing the expression of mRNA transcript or mRNA precursors, such as nascent RNA, of said gene. Said analysis can be assessed by preparing mRNA/cDNA from cells in the sample obtained from the patient, and hybridizing the mRNA/cDNA with a probe. The prepared mRNA/cDNA can be used in hybridization or amplification assays that include, but are not limited to, Southern or Northern analyses, polymerase chain reaction analyses, such as quantitative PCR (TaqMan), and probes arrays such as GeneChip(TM) DNA Arrays (AFF YMETRIX). Advantageously, the analysis of the expression level of mRNA transcribed from the gene encoding for ROBO4 involves the process of nucleic acid amplification, e. g., by RT-PCR (the experimental embodiment set forth in U. S. Patent No. 4,683, 202), ligase chain reaction (BARANY, Proc. Natl. Acad. Sci. USA, vol.88, p: 189-193, 1991), self sustained sequence replication (GUATELLI et al., Proc. Natl. Acad. Sci. USA, vol.57, p: 1874-1878, 1990), transcriptional amplification system (KWOH et al., 1989, Proc. Natl. Acad. Sci. USA, vol.86, p: 1173-1177, 1989), Q-Beta Replicase (LIZARDI et al., Biol. Technology, vol.6, p: 1197, 1988), rolling circle replication (U. S. Patent No. 5,854, 033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5 'or 3 'regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

In another preferred embodiment, the expression of level of ROBO4 in the sample may be assessed by determining the number of cancer cells that express ROBO4 and/or the level of ROBO4 at the surface of the cancer cells. Standard methods for detecting the expression of a specific surface marker at cell surface are well known in the art. Typically, the step consisting of measuring the level of ROBO4 at the cancer cell surface may consist in collecting the population of cancer cells and using at least one differential binding partner directed against the ROBO4, wherein the cancer cells are bound by the binding partners to ROBO4. As used herein, the term "binding partner directed against ROBO4" refers to any molecule (natural or not) that is able to bind ROBO4 with high affinity. Said binding partners include but are not limited to antibodies, aptamer, and peptides. The binding partners may be antibodies that may be polyclonal or monoclonal, preferably monoclonal, specifically directed against ROBO4.

Polyclonal antibodies of the disclosure or a fragment thereof can be raised according to known methods by administering the appropriate antigen or epitope to a host animal selected, e.g., from pigs, cows, horses, rabbits, goats, sheep, and mice, among others. Various adjuvants known in the art can be used to enhance antibody production. Although antibodies useful in practicing the disclosure can be polyclonal, monoclonal antibodies are preferred. Monoclonal antibodies or a fragment thereof can be prepared and isolated using any technique that provides for the production of antibody molecules by continuous cell lines in culture. Techniques for production and isolation include but are not limited to the hybridoma technique originally; the human B-cell hybridoma technique; and the EBV-hybridoma technique.

The binding partners of the disclosure such as antibodies or aptamers may be labelled with a detectable molecule or substance, such as preferentially a fluorescent molecule, or a radioactive molecule or any others labels known in the art. Labels are known in the art that generally provide (either directly or indirectly) a signal. As used herein, the term "labelled", with regard to the antibody or aptamer, is intended to encompass direct labelling of the antibody or aptamer by coupling (i.e., physically linking) a detectable substance, such as a fluorophore [e.g. fluorescein isothiocyanate (FITC) or phycoerythrin (PE) or Indocyanine (Cy5)]) or a radioactive agent to the antibody or aptamer, as well as indirect labelling of the probe or antibody by reactivity with a detectable substance. An antibody or aptamer of the disclosure may be labelled with a radioactive molecule by any method known in the art. For example radioactive molecules include but are not limited radioactive atom for scintigraphic studies such as 1123, 1124, In111, Re186, Re188. Preferably, the antibodies are already conjugated to a fluorophore (e.g. FITC-conjugated and/or PE-conjugated).

The aforementioned assays may involve the binding of the binding partners (ie. antibodies or aptamers) to a solid support. The solid surface could a microtitration plate coated with the binding partner. After incubation of the sample, cancer cells that express ROBO4 specifically bound to the binding partner. Alternatively, the solid surfaces may be beads, such as activated beads, magnetically responsive beads. Beads may be made of different materials, including but not limited to glass, plastic, polystyrene, and acrylic. In addition, the beads are preferably fluorescently labelled. In a preferred embodiment, fluorescent beads are those contained in TruCount(TM) tubes, available from Becton Dickinson Biosciences, (San Jose, California). According to the disclosure, methods of flow cytometry are preferred methods for measuring the level of ROBO4 at the cell surface. Said methods are well known in the art. For example, fluorescence activated cell sorting (FACS) may be therefore used.

In one embodiment, the predetermined reference values may be index values or may be derived from one or more risk prediction algorithms or computed indices for bone metastases development. A predetermined reference value can be relative to a number or value derived from population studies. Such predetermined reference values can be derived from statistical analyses and/or risk prediction data of populations obtained from mathematical algorithms and computed indices of metastases development. In one embodiment of the present disclosure, the predetermined reference value is derived from the expression level of ROBO4 in a control sample derived from one or more subjects who did not develop bone metastases. In another embodiment, such subjects are monitored and/or periodically retested for a diagnostically relevant period of time ("longitudinal studies") following such test to verify continued absence of bone metastases. Such period of time may be one year, two years, two to five years, five years, five to ten years, ten years, or ten or more years from the initial testing date for determination of the reference value. Furthermore, retrospective measurement of the expression level of ROBO4 in properly banked historical subject samples may be used in establishing these predetermined reference values, thus shortening the study time required. Typically, the levels of ROBO4 in a patient who is at risk of having bone metastases is deemed to be higher than the reference value obtained from patient who never develop bone metastases.

The invention will be further illustrated by the following figures and examples. However, these examples and figures should not be interpreted in any way as limiting the scope of the present invention.

### FIGURES:

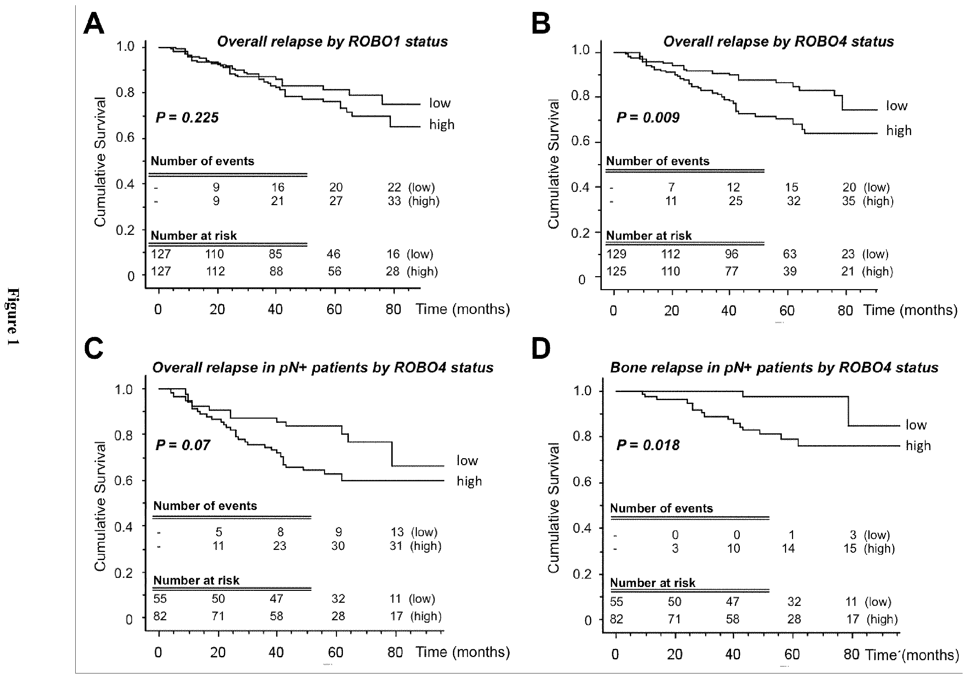

**Figure 1****:** ROBO4 expression in primary breast tumors is associated with worse prognosis in relapse-free survival. (A) Kaplan-Meier estimates for rates of relapse-free survival in 254 patients with breast cancer, according to ROBO1 status. (B) same as in (A), but cases are categorized according to ROBO4 status. (C) rates of relapse-free survival in 137 lymph node-positive (pN+) breast cancer patients, according to ROBO4 status. (D) same as in (C), but for bone metastasis-free survival.

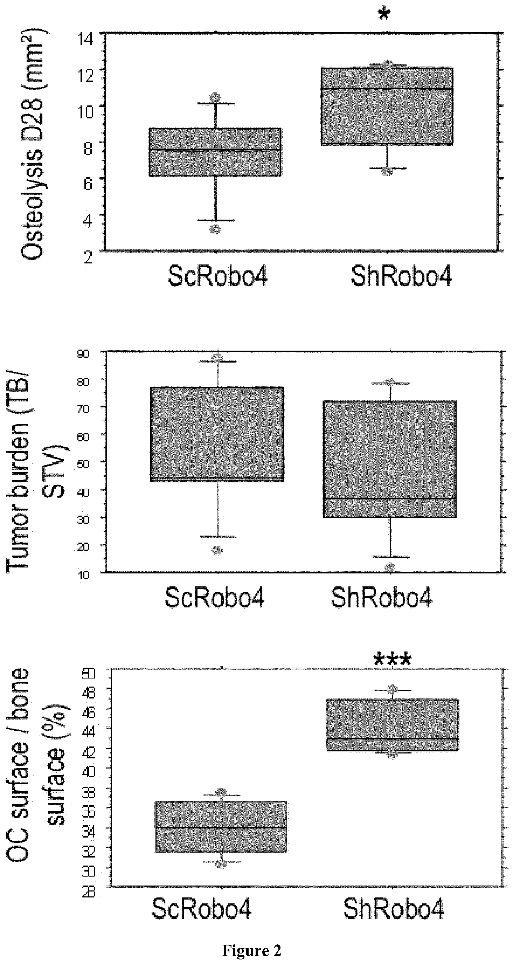

**Figure 2****:** Sh- or Sc-Robo4 transfectants were inoculated into the tail artery of animals. On day 28 after tumor cell inoculation, bone lesions were analyzed by radiography, histology and TRAP staining. Representative images of bone lesions in each group are shown on the left-hand side. Quantitative data are shown on the right-hand side.

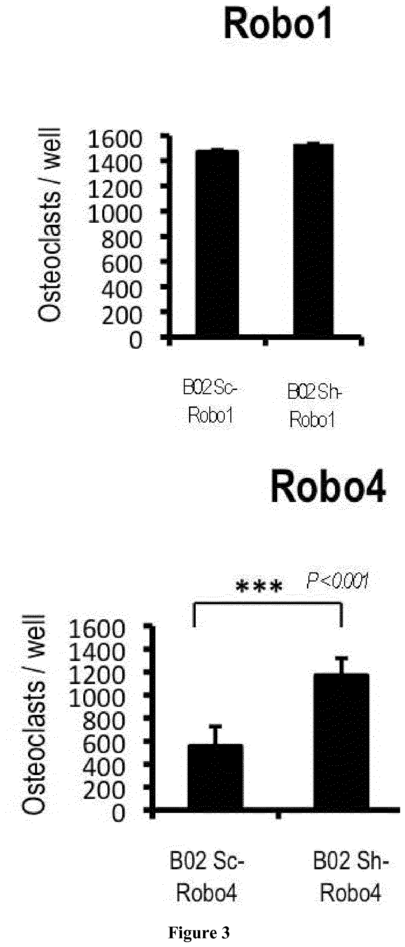

**Figure 3****:** *In vitro* osteoclast formation of murine bone marrow cells treated with MCSF+RANKL and the conditioned medium from breast cancer cells silenced for ROBO1 or ROBO4. Mature osteoclasts were quantified as multinucleated, TRAP-positive cells. Representative images of mature osteoclasts are shown for each group.

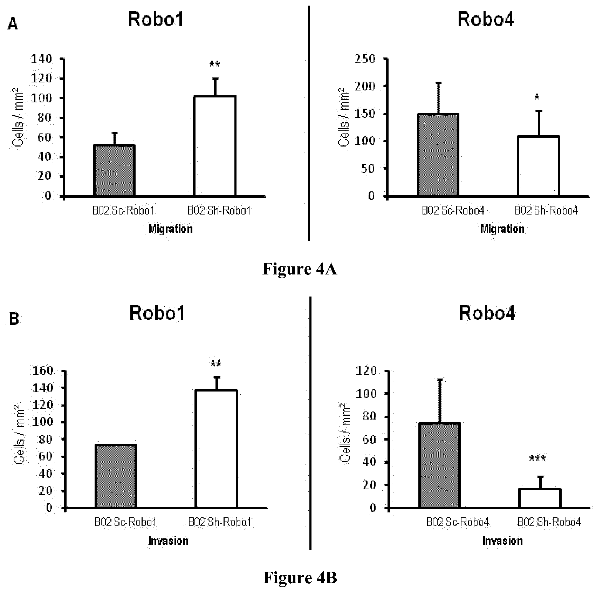

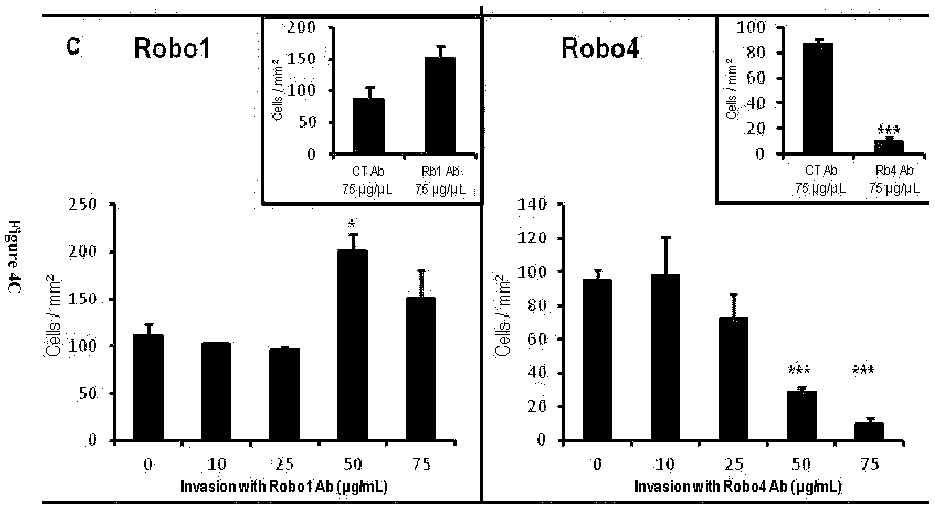

**Figure 4****:** *(A)* Cell migration assay. Breast cancer cells silenced for ROBO1 (left side) or ROBO4 (right side) were loaded in inserts with a porous membrane (upper chamber) and the chemoattractant (serum) was placed in wells of a companion plate (lower chamber). After 6-h incubation at 37°C, the nonmigrating cells were removed and the migrating cells on the under surface of the inserts were fixed, stained and counted under microscope. (B) Cell invasion assay. For cell invasion, inserts were coated with basement membrane Matrigel. After 24-h incubation at 37°C, noninvading cells were removed and remaining of the experiments was performed as described in (A). (C) Effects of increasing concentrations of anti-Robo1 (left) or anti-Robo4 antibody (right) on invasion of osteotropic B02 breast cancer cells. *Insets*: effects of the highest concentrations of anti-Robo1 and anti-Robo4 antibodies compared with isotype-matched negative control antibodies.

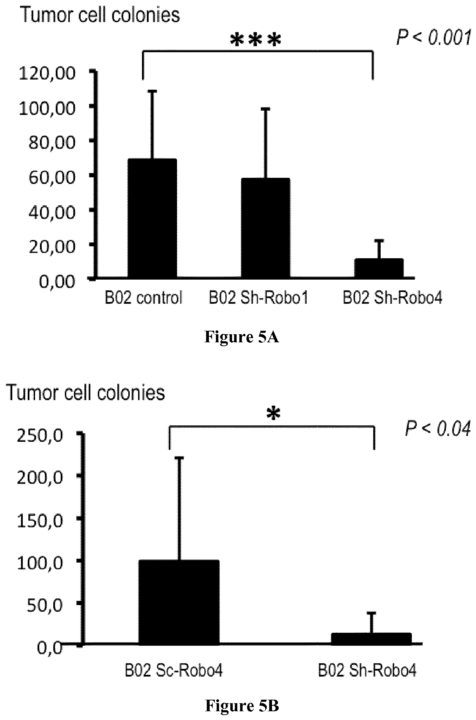

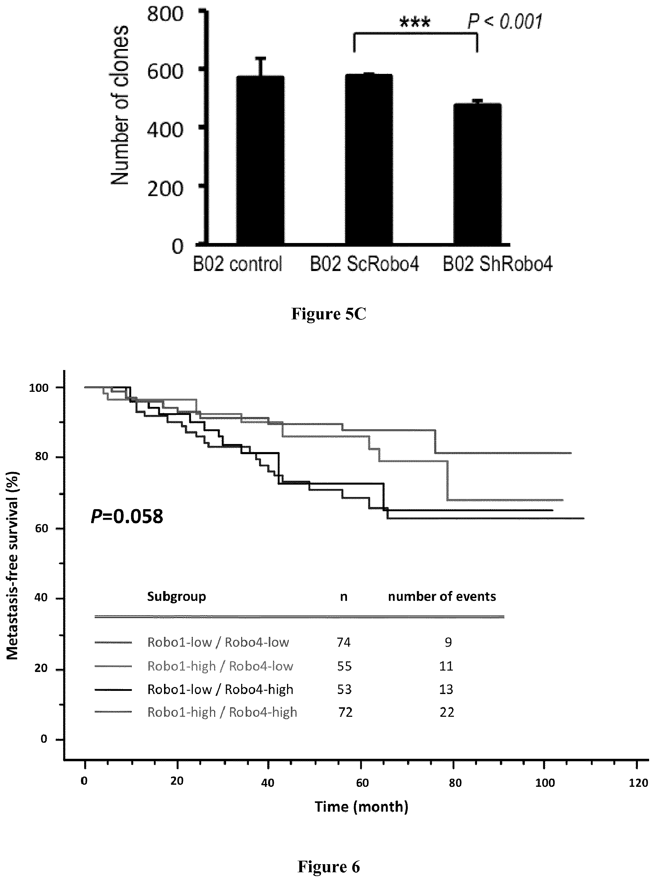

**Figure 5****:** *(A)* Seven days after intra-arterial inoculation of Sh-Robo1/4 transfectants or parental B02 breast cancer cells, animals were culled, the hind limbs collected and the bone marrow flushed, as depicted in the drawing. Cultured bone marrow cells were placed under antibiotic selection, allowing the selective growth of antibiotic-resistant tumor cells. Colonies of tumors cells were fixed, stained, and counted. Representative images for each group are shown. (B) same as in (A), but for intratibial inoculation of Sc- and Sh-Robo4 transfected tumor cells. (C) soft agar colony formation assay. ROBO4 silencing decreases the number and size of colonies.
**Figure 6****:** Kaplan-Meier estimates for rates of relapse-free survival in 254 patients with breast cancer, according to ROBO1 and ROBO4 status. P = 0.058 by log-rank test. The total number of metastasis events (number of events) in each subgroup is shown in the embedded table.

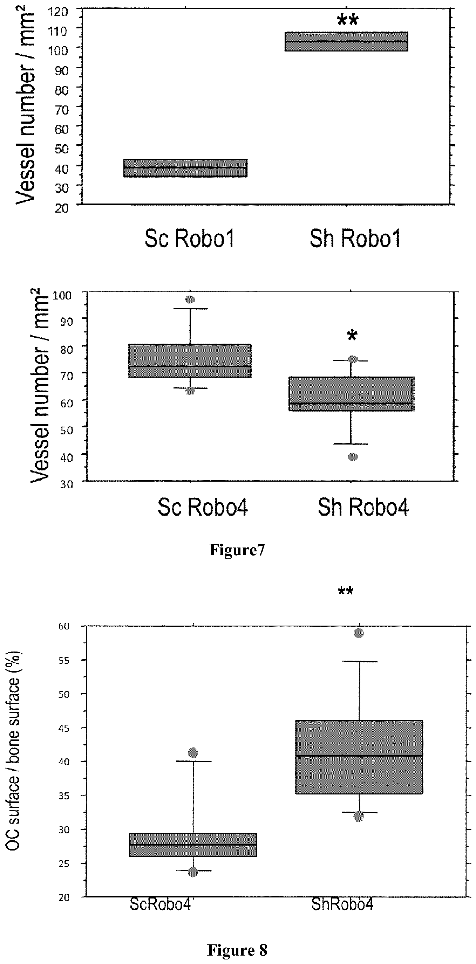

**Figure 7****:** Association between tumor angiogenesis and the silencing of ROBO1 or ROBO4 in tumor cells. *(A)* Immunohistochemical analysis of Sc-ROBO1 and Sh-ROBO1 tumor xenografts on day 50 after tumor cell inoculation, using an anti-CD31 antibody that specifically recognizes murine endothelial cells. The number of CD31-positive blood vessels within tumors was quantified. Arrows show blood vessels. ** *P* < 0.01. (B) Same as in (A), but for Sc-ROBO4 and Sh-ROBO4 tumor xenografts. * *P* < 0.05.
**Figure 8****:** In situ detection of osteoclasts following tartrate-resistant acid phosphatase (TRAP) staining of tibial metaphysis of metastatic legs from animals inoculated intratibially with Sh- or Sc-Robo4 transfected tumor cells. Representative images are shown on the left. Osteoclasts are stained red (arrow). The osteoclast (OC) surface relative to bone surface ratio was expressed in percentage. **, P < 0.01 compared with Sc-Robo4 group.

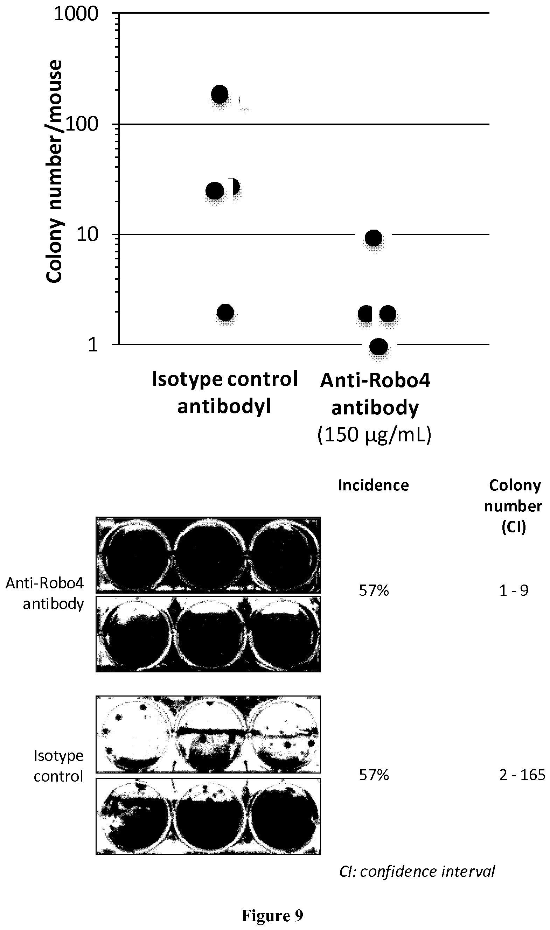

**Figure 9****.** The treatment of human B02 breast cancer cells with a polyclonal antibody directed against Robo4 inhibits the formation of bone marrow micrometastases in animals.

### EXAMPLE 1:

### Material & Methods

### Patients

Clinical and biological characteristics of the cohorts of patients with breast cancer have been described elsewhere (Berthier et al., 2010). Median follow-up of the cohorts of patients from Hospices Civils de Lyon (n = 254) and René Hughenin hospital (n = 456) were 54 and 120.5 months, respectively.

### Real-time RT-PCR

Total RNA from human breast tumors and murine bone marrow was extracted using Trizol reagent (Sigma). To remove any genomic DNA contamination, total RNA was treated with RNAse-free DNAse I and purified using RNeasy microcolumns (Qiagen). RNA quality was verified using an Agilent Bioanalyser 2100 (Agilent Technologies). cDNA was synthesized using iScript cDNA Synthesis kit (Biorad). PCR reactions were run using SYBR® Green qPCR kit (Life technologies) in 96-well plates on a Mastercycler EP system (Realplex2, Eppendorf, hamburg-Eppendorf, Germany) according to the manufacturer's instructions. Real-time RT-PCR was carried out with an initial step for 2 minutes at 95°C followed by 40 cycles of 15 seconds at 95°C, 15 seconds at Tₘ (mL32: 58°C, mSlit2: 64°C, TBP: 67°C, Robo1: 63°C, Robo4: 67°C), and 20 seconds at 72°C. ROBO1 and ROBO4 expression were normalized with TBP. Murine Slit2 was normalized with mL32.

### Cell lines, cell culture and transfection.

The human B02 breast cancer cell line is a subpopulation of the MDA-MB-231 cancer line that was selected for the high efficiency with which it metastasizes to bone in animals (Peyruchaud et al., 2003). A Flp-in-expressing subclone (MDA-MB-231/B02-Frt11), which has been previously generated (Buijs et al., 2007), was used here for cell transfection experiments.

Small hairpin RNAs (ShRNA) directed against human ROBO1 mRNA and corresponding scrambled sequences (ScRNA) were designed with the Si Designer Tool (Promega). The oligonucleotides used were as follows: ShRNA forward, 5'-ACCgCAgTACTAAgggAA-CAATAAgTTCTCTATTgTTCCCTTAgTACTgCTTTTTC-3'; ShRNA reverse, 5'-TgCAgAAAAAgCAg-TACTAAgggAACAATAgAgAACTTATTgTTCCCTT-AgTACTg-3'. The shRNAs and corresponding scrambles duplex were synthesized with the psiSTRIKE puromycin kit (Promega) under U6 promoter. Plasmids were transfected into B02-Frt cells using the Transfast reagent (Promega). Cells were cultured for 2 weeks in presence of puromycin (2 µg/mL, PAA) and clones were isolated using cloning cylinders.

ShRNA targeting ROBO4 mRNA and their corresponding control ScRNA, were designed with the siRNA design tools (Genscript) and cloned into the pRNA-U6.1/zeocin vector (Genscript). The oligonucleotides used were as follows: ShRNA forward, 5'-GAGCAGAGAAGAGTGACGA-3'; ShRNA reverse, 5'-TCTTACACGGCCTTGTTCA-3'. Following transfection, cells were cultured for 3 weeks in the presence of zeocin (800 µg/mL, Life technologies), and clones were then isolated using cloning cylinders. Clones silenced for ROBO1 or ROBO4 and their respective controls were then selected by western blotting.

### Western blotting

Protein cell extracts were electrophoresed on a 4-12% gradient SDS-polyacrylamide gel (Life technologies), then transferred onto nitrocellulose membranes (Millipore, Billerica, MA). Antibodies against Robo1 (Ab7279; AbCam; 1:300 dilution), Robo4 (Ab10547, AbCam; 1:500 dilution), a-tubulin (Sigma; 1:2,000 dilution), Akt (#9272, Cell Signaling; 1:2,000 dilution), phospho-AkT (#9271S, Cell signaling; 1:2,000 dilution), Src (clone GD11, Millipore; 1:500 dilution), phospho-Src (clone 9A6, Millipore; 1:500 dilution), and cyclin D1 (clone EPR2241, Millipore; 1:10,000 dilution) were used for immunoblotting, according to manufacturers' instructions. After incubation with primary antibodies, membranes were incubated with horseradish peroxide (HRP)-conjugated donkey anti-rabbit and anti-mouse secondary antibodies (Amersham; 1:2,000 dilution), and immunostaining was then performed with enhanced chemiluminescence (ECL) detection system (Perkin Elmer).

### Cell migration and invasion assays.

Experiments were conducted in 24-well cell culture plates with 8-µm diameter pore-size inserts, as previously described (Zhang et al., 2007; Fradet et al., 2011). For cell invasion assays, inserts were coated with 100 µl basement membrane Matrigel (0.3 mg/ml). Parental tumor cells or transfectants (1.6 X 10⁵ cells/ml) were re-suspended in culture medium containing 0.1% (w/v) bovine serum albumin and 300 µl of this cell suspension were loaded into each insert (upper chamber). For the experiments with anti-Robo1 (clone 1F8, Sigma) and anti-Robo4 (ab10547, Abcam) antibodies, each of these antibodies was preincubated 90 min at 37°C with tumor cells before adding to the upper chambers. The chemoattractant [10% (v/v) fetal calf serum] was placed in the lower chamber (750 µl /well). For cell migration and invasion experiments, plates were incubated for 6 h and 24h at 37°C in a 5% CO₂ incubator, respectively. After incubation, inserts were collected, the nonmigrating cells were removed and the migrating cells on the under surface of the inserts were fixed and stained with crystal violet. Cells were then counted under microscope.

### Animals

Four-week-old female NMRI and Balb/c immunocompromised mice and OF1 male mice were purchased from Charles River Laboratories (St. Germain sur l'Arbresle, France). All procedures involving animals, including their housing and care, the method by which they were culled, and experimental protocols were conducted in accordance with a code of practice established by the local ethical committee of the University of Lyon.

### Animal studies

Orthotopic tumor xenograft experiments were conducted in NMRI immunodeficient mice, as previously described (Fradet et al., 2011). Four-week-old female nude mice were injected in the fat pad of the 4^{th} mammary gland with a pool of transfectants (10⁶ cells in 50-µL PBS). At the end of the protocols, mice were sacrificed and tumors collected, weighted then prepared for immunohistochemistry.

Intraosseous tumor xenograft experiments were conducted in female Balb/c nude mice, as previously described (Zhang et al., 2007). Briefly, a small hole was drilled with a 30-gauge sterile needle through the tibial plateau with the knee flexed. Using a new sterile needle fitted to a 50 ml-sterile Hamilton syringe, a single-cell suspension (10⁵ cells in 30-µl PBS) was injected in the bone marrow cavity. The progression of osteolytic lesions was monitored by radiography of anesthesized animals, using a cabinet X-ray system.

Bone metastasis experiments were conducted in female Balb/c nude mice, as described previously (Fradet et al., 2011; Peyruchaud et al., 2003; Zhang et al., 2007). Transfectants (5 X 10⁵ cells in 100-µL PBS) were injected into the tail artery of anesthetized nude mice. Radiographs were taken as described above. Osteolytic lesions were identified on radiographs as radiolucent lesions in the bone. The area of osteolytic lesions was measured using a computerized image analysis system, and the extent of bone destruction per leg was expressed in square millimeters.

### Bone histology, histomorphometry and immunohistochemistry

Bone histology and histomorphometric analysis of bone tissue sections were performed as previously described (Fradet et al., 2011; Peyruchaud et al., 2003; Zhang et al., 2007). Histomorphometric measurements [bone volume (BV)/tissue volume (TV) and tumor volume (TuV)/soft tissue volume (STV) ratios] were performed in a standard zone of the tibial metaphysis, situated at 0.5 mm from the growth plate, including cortical and trabecular bone. The BV/TV ratio represents the percentage of bone tissue. The TuV/STV ratio represents the percentage of tumor tissue. The in situ detection of osteoclasts was performed on tartrate-resistant acid phosphatase (TRAP)-stained longitudinal paraffin-embedded medial sections of tibial metaphysis with the use of a commercial kit (Sigma). Osteoclast resorption surface was calculated as the ratio of TRAP-positive trabecular bone surface to the total trabecular bone surface at the the tumor-bone interface. For blood vessel immunodetection, tumor sections were incubated with a rabbit polyclonal anti-CD31 antibody (AnaSpec) followed by an anti-rabbit HRP-linked secondary antibody. Tumor microvessel density was quantified, as previously described (Fradet et al., 2011; Peyruchaud et al., 2003).

### Osteoclastogenesis assay

Experiments were conducted as described previously (Fradet et al., 2011). Briefly, bone marrow cells from hind limbs of 6-week-old OF1 male mice were cultured in α-MEM medium containing 10% (v/v) fetal calf serum supplemented with M-CSF (20 ng/ml) and RANK-L (200 ng/ml), in the presence of conditioned medium from transfectants (20 µg/mL). After 7 days, mature osteoclasts were enumerated under a microscope on the basis of the number of nuclei (more than three nuclei) and TRAP activity. Results were expressed as the number of osteoclasts per well.

### Ex-vivo bone marrow micrometastasis assay.

Sh/Sc-Robo transfectants and parental B02 breast cancer cells were injected into the tail artery of animals and animals were culled on day 7 after tumor cell inoculation. Hind limbs were collected, and both ends of tibiae and femurs were cut and the bone marrow flushed with culture medium using a 23-gauge needle. Bone marrow cells were then seeded in 6-well plates and cultured in complete medium. After 1-day culture, bone marrow cells were placed under antibiotic selection for 2 weeks, allowing the selective growth of antibiotic-resistant tumor cells. Colonies of tumors cells were fixed, stained with crystal violet, and counted.

### Cytokine array

A commercial antibody-based protein microarray designed to detect 79 growth factors, cytokines, and chimiokines (RayBio Human Cytokine Array V, RayBiotech) was used. Array membranes were incubated for 2 h with the conditioned medium from cultured transfected cells (150 µg/mL). After washing, membranes were incubated with a cocktail of 79 biotinylated antibodies, and the rest of the experimental procedure was carried out following manufacturer's instructions.

### Statistical analysis

All data were analyzed using StatView software (version5.0; SAS Institute Inc, Cary, NC). Results are reported as mean ± SD, as indicated in the figure legends and tables. Regarding preclinical studies, pairwise comparisons were carried out by performing a nonparametric Mann-Whitney U test. Regarding clinical data, the distribution of Robo1 and Robo4 expression in relation to the usual prognostic parameters was carried out using the Mann-Whitney or Kruskall-Wallis test. Comparison between Kaplan-Meier curves were performed using the log-rank test. P values less than 0.05 were considered statistically significant.

### Results

### Association of ROBO1lROBO4 expression with clinical relapse of breast cancer.

We previously selected a subpopulation of the MDA-MB-231 human breast cancer cell line (referred to as MDA-MB-231/B02) that only metastasizes to bone (Peyruchaud et al., 2003). Using A and B U133 Affymetrix GeneChips, we have compared the gene-expression profiles of MDA-MB-231 and MDA-MB-231/B02 cells and found that MDA-MB-231/B02 cells express a bone-metastasis gene signature (Bellahcène et al., Breast Cancer Res Treat., 101:135-148, 2007; Garcia et al., Clin Exp Metastasis, 25:33-42, 2008), which includes genes from the ROBO/SLIT family. To be more precise, osteotropic MDA-MB-231/B02 cells overexpressed ROBO1 and ROBO4 compared with the parental cell line. ROBO2 and ROBO3 were not expressed by MDA-MB-231 and MDA-MB-231/B02 cells. The Robo ligands SLIT2 and, to a lesser extent, SLIT1 (but not SLIT3) were also expressed by MDA-MB-231 and MDA-MB-231/B02 cells.

To investigate the association of ROBO1 and ROBO4 expression with breast cancer outcome, we examined a cohort of 254 patients for whom there was no evidence of distant metastasis at the time of diagnosis and no previous treatment (Berthier et al., 2010). We did not observe any correlation between ROBO1 status and clinical and biological characteristics in breast cancer patients (Table 1). In striking contrast, there was a statistically significant correlation between ROBO4 expression and the node status (P = 0.001); high ROBO4 levels in primary tumors being more frequently observed with patients having more than three positive lymph nodes (Table 1). To define the prognostic values of ROBO1 and ROBO4 expression in breast cancer patients, survival curves were estimated using the Kaplan-Meier method. Rates of relapse-free survival did not differ significantly between groups expressing high or low levels of ROBO1. Conversely, the number of relapses at a median follow-up of 54 months in the group with high ROBO4 expression levels was twice as high as in the group with low ROBO4 levels (32 *versus* 15 relapses, respectively; P = 0.009 by the log-rank test).

Additionally, subgroup analysis of the cohort of patients according to ROBO1 and ROBO4 status showed that relapses occurred more frequently in patients having primary tumors with high ROBO4 levels, regardless of whether ROBO1 expression levels were high or low (P = 0.058). Because lymph-node status has been previously correlated with a high risk of metastasis in the cohort we analyzed (Berthier et al., 2010) and a significant correlation between ROBO4 expression and the node status was observed here (Table 1), we verified whether the node status could be a confounding factor. In lymph-node negative (pN0) patients, ROBO4 expression was not related to risk of metastasis (not shown). Conversely, when the analysis was restricted to lymph-node positive (pN+) patients, high ROBO4 levels correlated with distant relapses (P = 0.07 by log-rank test) and bone relapses (P = 0.018 by log-rank test). Thus, a high ROBO4 expression correlated with worse prognosis.

### ROBO1 silencing enhances, whereas ROBO4 silencing reduces experimental primary tumor growth

To determine whether there is a link between ROBO1/ROBO4 expression and tumor growth within the mouse mammary gland, we stably reduced the endogenous levels of ROBO1 or ROBO4 in human MDA-MB-231/B02 breast cancer cells using RNA interference strategy. shRNA-mediated silencing reduced Robo1 protein levels by > 90%, when compared with that observed in scrambled-transfected ScRobo1 and MDA-MB-231/B02 parental cells. Robo4 levels remained unchanged in ShRobo1 and ScRobo1 breast cancer cells. A pool of MDA-MB-231/B02 transfectants silenced for ROBO1 (clones Sh1.32 and Sh1.33) and a pool of mock-transfectants (clones Sc2.1 and Sc2.4) were then implanted orthotopically within the mouse mammary gland of immunodeficient mice. Analysis of the median primary tumor size demonstrated that ShRobo1 tumors were larger than ScRobo1 tumors. Immunohistochemical analysis of tumor xenografts with an anti-CD31 antibody that specifically recognizes murine endothelial cells showed that ROBO1 silencing led to a substantial increase of microvessel density in ShRobo1 tumors compared with ScRobo1 tumors. VEGF levels produced by ShRobo1 breast cancer cells were also statistically significantly increased compared with ScRobo1 and parental B02 cells (data not shown). In sharp contrast, the orthotopic implantation within the mouse mammary gland of B02 breast cancer cells silenced for ROBO4 led to a 3-fold decrease in median size of primary ShRobo4 tumors compared with ScRobo4 tumors. The smaller size of ShRobo4 tumors was associated with decreased tumor angiogenesis, as judged by CD31 immunostaining. VEGF levels produced by ShRobo4 and ScRobo4 breast cancer cells were however similar. Taken together, these data suggested that Robo1 and Robo4 expressed on tumor cells had antagonistic functions; Robo1 and Robo4 being, respectively, negative and positive regulators of primary tumor growth.

### Robol and Robo4 differently regulate breast cancer skeletal outgrowth and the formation of osteolytic lesions.

To test the role of Robo1 and Robo4 in bone metastasis, a pool of MDA-MB-231/B02 transfectants silenced for ROBO1 (clones Sh1.32 and Sh1.33) or a pool of transfectants silenced for ROBO4 (clones Sh2.6 and Sh4.5) or pools of the respective mock-transfectants for ROBO1 (clones Sc2.1 and Sc2.4) and ROBO4 (clones Sc1.2 and Sc2.2) were injected into the tail artery of immunodeficient mice. Using this animal model, radiographic analysis on day 28 after tumor cell injection revealed that the extent of osteolytic lesions in hind limbs of animals bearing ShRobo1 tumors was increased, being 40% larger than that of animals bearing mock-transfected ScRobo1 tumors. This difference was accompanied with a sharp reduction of the BV/TV ratio (indicating a higher bone destruction) and a 3-fold increase in the TB/STV ratio (a measure of the skeletal tumor burden) compared with mice bearing mock-transfected ScRobo1 tumors, as determined by histomorphometric examination. Silencing of ROBO4 led to a modest increase in the extent of osteolytic lesions in ShRobo4 tumor-bearing animals compared with mice bearing mock-transfected ScRobo4 tumors. However, histomorphometric analysis of hind limbs with metastases showed that the BV/TV and TB/STV ratios did not differ statistically significantly among the two groups of animals. Interestingly, tartrate-resistant acid phosphatase (TRAP) staining of bone tissue sections of metastatic legs from mice bearing ShRobo4 tumors showed that the active-osteoclast resorption surfaces at the tumor-bone interface were substantially increased compared with ScRobo4 tumors. By contrast, active-osteoclast resorption surfaces in metastatic legs from mice bearing ShRobo1 or ScRobo1 tumors were similar. These observations pointed to a specific role of tumor-derived Robo4 in regulating osteoclast-mediated bone destruction, whereas Robo1 expressed on tumor cells was associated with metastatic outgrowth in the skeleton.

To directly test whether the silencing of ROBO4 (or ROBO1) in tumor cells could influence osteoclast formation, we treated primary mouse bone marrow cell cultures with RANKL and macrophage colony-stimulating factor, which are two hematopoietic factors both necessary and sufficient to induce osteoclastogenesis, together with the conditioned medium of the different transfectants. Consistent with *in vivo* data, the conditioned media of Sh-Robo1 and Sc-Robo1 B02 breast cancer cells stimulated the formation of TRAP-positive multinucleated osteoclasts to a similar extent. Conversely, the conditioned medium of Sh-Robo4 B02 cells induced a 2-fold increase in osteoclast formation compared with that of Sc-Robo4 cells. We then used a human cytokine antibody array to measure cytokines in the conditioned medium from transfectants. Several cytokines and interleukins known to stimulate osteoclast activity (MCP-1, GM-CSF, P1GF, VEGF, IL-6, IL-8) were produced by these transfectants. Cytokine profiles were similar for Sc- and Sh-Robo1 cells. By contrast, there was a higher production of P1GF, MCP-1 and IL-8 by Sh-Robo4 cells than Sc-Robo4 cells, as determined by cytokine array and ELISA. These cytokines were also detected *in vivo.* In addition, concentrations of P1GF and IL-8 (but not MCP-1) were higher in the serum from animals bearing Sh-Robo4 tumors than that of the control group. Therefore, the enhanced active-osteoclast resorption surfaces in metastatic legs from animals bearing ShRobo4 tumors were likely the result of a higher production of pro-osteoclastic cytokines by tumor cells *in vivo.* Conversely, the larger osteolytic lesions in animals bearing Robo1-deficient tumors were not directly related to the silencing of ROBO1 but an indirect result of the greater number of ShRobo1 tumor cells residing in the bone marrow that, in turn, stimulated osteoclast-mediated bone destruction.

### Effects of ROBO1 and ROBO4 silencing or targeting by antibodies on breast cancer cell migration and invasion

In light of these results, we postulated that the silencing of ROBO1 could result of a faster bone colonization by ShRobo1 tumor cells. Indeed, there is some evidence in the literature that Slit-Robol regulates cancer cell migration (Mehlen et al., 2011). We therefore investigated whether Robo1 and/or Robo4 could regulate the migration/invasion of breast cancer cells, using a modified Boyden chamber assay. We first tested the effects of ROBO1 or ROBO4 silencing on the chemotactic response of breast cancer cells to serum. There was a 1.5- to 2-fold increase in the migration of Sh-Robo1 transfected cells (pool of clones Sh1.32 and Sh 1.33) compared with that of scrambled-transfected ScRobo1 cells (pool of clones Sc2.1 and Sc2.4). Similarly, there was a substantial gain in invasion of ShRobo1 cells when compared with ScRobo1 cells. In contrast, ROBO4 silencing led to a statistically significant decrease of ShRobo4 cell migration (pool of clones Sh2.6 and Sh4.5) compared with that of control cells (pool of clones Sc1.2 and Sc2.2). Additionally, there was a 4-fold decrease in invasion of ShRobo4 cells compared with that of ScRobo4 cells. Next, we evaluated the benefit of targeting Robo1 or Robo4 with antibodies on invasion of parental B02 breast cancer cells. Compared with isotype-matched control antibodies, the treatment of B02 breast cancer cells with a monoclonal anti-Robo1 antibody promoted invasiveness, whereas B02 cell invasion was dose-dependently inhibited in the presence of increasing concentrations of a polyclonal anti-Robo4 antibody. Taken together, we concluded that Robo1 and Robo4 in tumor cells have anti-invasive and pro-invasive properties, respectively.

These results suggested therefore that Robo1 silencing could draw breast cancer cells to colonize bone faster when injected intravenously, thereby explaining the higher skeletal tumor burden in legs from animals bearing Robo1-deficient tumors. This assumption was indeed supported by the observation that, when Sc-Robo1 and Sh-Robo1 transfected cells were directly inoculated into the tibial bone marrow cavity, mice bearing control or Robo1-depleted tumors had a similar extent of bone destruction and skeletal tumor burden, as determined by radiography and histomorphometric examination. Thus, ROBO1 silencing endowed tumor cells with a higher invasiveness *in vivo.* Regarding Robo4 function in bone metastasis, osteolytic lesions formed by Sh-Robo4 tumor cells were comparable to that observed in metastatic legs from animals bearing Sc-Robo4 tumors. This was unexpected given that ROBO4 silencing decreased the skeletal tumor burden in metastatic animals, compared with ScRobo4 tumors. However, there was also a sharp increase of TRAP-positive osteoclasts at the tumor-bone interface and higher levels of the pro-osteoclastic cytokine IL-8 in the serum of animals bearing ShRobo4 tumors, compared with ScRobo4 tumors. Thus, a high endogenous osteoclast-stimulatory activity of ShRobo4 tumor cells likely explained why ShRobo4 tumor-bearing animals had osteolytic lesions despite a reduction of skeletal tumor burden.

### ROBO4 silencing reduces the survival and outgrowth of breast cancer cells in the bone marrow

Tumor cell invasion occurs at a very early stage during bone metastasis formation, enabling tumor cells to seed and thrive in the bone marrow (Weilbaecher et al., 2011). Because Robo1 and Robo4 were regulating breast cancer cell invasion *in vitro* and bone metastasis formation *in vivo,* we hypothesized that these receptors might facilitate the engraftment of tumor cells in the bone marrow microenvironment. To address this question, Sh-Robo transfectants and parental B02 breast cancer cells were injected into the tail artery of animals and these animals were then culled on day 7 after tumor cell inoculation, at which time there is no evidence of metastases, as judged by radiography, bioluminescence imaging and histology (Garcia et al., 2008). The bone marrow was then flushed from the hind limbs of these animals and placed in culture under antibiotic selection, allowing the selective growth of antibiotic-resistant tumor cells. Compared with mice inoculated with parental B02 cells or ShRobo1 cells, the number of tumor cell colonies in the bone marrow from mice inoculated with ShRobo4 cells was reduced by 75 to 80%. Morever, when directly inoculated into the bone marrow cavity of tibiae and tracked for tumor outgrowth, the number of ShRobo4 colonies in the bone marrow was reduced by 78% compared with that observed for ScRobo4 cells. A similar effect was observed when tumor cells were grown anchorage-independently in soft agar. Compared with ScRobo4 and parental B02 cells, the number and size of colonies formed by ShRobo4 cells were statistically significantly decreased. Thus, Robo4 was required for the survival of breast cancer cells in the bone marrow.

### EXAMPLE 2:

B02 cells pre-treated with anti-Robo4 antibody were injected into the tail artery of immunodeficient mice. One week after tumor cell inoculation, the bone marrow was flushed from the hind limbs of these animals and placed in culture under antibiotic selection, allowing the selective growth of antibiotic-resistant tumor cells. After 2 weeks in culture, tumor cell colonies were fixed, stained and counted. Each series of 3 wells shows outgrowth of tumor cell colonies from the bone marrow of a single animal. There were 4 mice per group. Figure 9 shows that the treatment of human B02 breast cancer cells with a polyclonal antibody directed against Robo4 inhibits the formation of bone marrow micrometastases in animals. A PCR analysis shows that while most of the breast cancer cell lines expressed Robo1, only those having a bone tropism (B02 and BC-M1) expressed Robo4. Additionally, it was found that Hs578T and T47D cells strongly expressed Robo2.

**Table 1**

| Characteristics | | | Robo1 expression | | | Robo4 expression | | |
|---|---|---|---|---|---|---|---|---|
| | | n | low ^{(a)} | high ^{(a)} | *P* | low ^{(a)} | high ^{(a)} | *P* |
| Menopausal status | Pre | 147 | 77 | 70 | .*45* | 82 | 65 | .*08* |
| | Post | 107 | 50 | 57 | | 47 | 60 | |
| Tumor size | < 20 mm | 103 | 60 | 43 | .*05* | 52 | 51 | .*99* |
| | > 20 mm | 143 | 64 | 79 | | 73 | 70 | |
| Histological grade | 1 | 27 | 15 | 12 | .*74* | 15 | 12 | .*12* |
| | 2 | 110 | 52 | 58 | | 52 | 58 | |
| | 3 | 58 | 28 | 30 | | 37 | 21 | |
| Node status | negative | 117 | 66 | 51 | .*13* | 74 | 43 | .*001* |
| | 1-3 | 83 | 35 | 48 | | 39 | 44 | |
| | >3 | 54 | 26 | 28 | | 16 | 38 | |
| ER status | negative | 42 | 15 | 27 | .*06* | 22 | 20 | .*95* |
| | positive | 212 | 112 | 110 | | 107 | 105 | |
| PR status | negative | 55 | 26 | 29 | .*76* | 30 | 25 | .*63* |
| | positive | 199 | 101 | 98 | | 99 | 100 | |

| | | | | | | | | |
|---|---|---|---|---|---|---|---|---|
| ^{(a)} low: < 50% quartile; high: > 50% quartile. P values are for comparaisons between high and low groups using the Chi-2 test. | | | | | | | | |

### REFERENCES:

Alajez, N.M., Lenarduzzi M, Ito E, Hui AB, Shi W, Bruce J, Yue S, Huang SH, Xu W, Waldron J, et al. (2011) MiR-218 suppresses nasopharyngeal cancer progression through downregulation of survivin and the SLIT2-ROBO1 pathway. Cancer Res 71, 2381-2391.
Bellahcène, A., Bachelier, R., Detry, C., Lidereau, R., Clézardin, P., and Castronovo, V. (2007) Transcriptome analysis reveals an osteoblast-like phenotype for human osteotropic breast cancer cells. Breast Cancer Res Treat 101, 135-148.
Berthier, A., Seguin, S., Sasco, A.J., Bobin, J.Y., De Laroche, G., Datchary, J., Saez, S., Rodriguez-Lafrasse, C., Tolle, F., Fraichard, A., et al. (2010) High expression of gabarapl1 is associated with a better outcome for patients with lymph node-positive breast cancer. Br J Cancer 102, 1024-1031.
Buijs, J.T., Henriquez, N.V., van Overveld, P.G., van der Horst, G., Que, I., Schwaninger, R., Rentsch, C., Ten Dijke, P., Cleton-Jansen, A.M., Driouch, K., Lidereau, R., et al. (2007) Bone morphogenetic protein 7 in the development and treatment of bone metastases from breast cancer. Cancer Res 67, 8742-8751.
Chang, P-H., Hwang-Verslues, W.W., Chang, Y-C., Chen, C-C., Hsiao, M., Jeng, Y-M., Chang, K-J., Lee, E. Y., Shew, J-Y., and Lee, W-H. (2012) Activation of Robo1 signaling of breast cancer cells by Slit2 from stromal fibroblast restrains tumorigenesis via blocking PI3K/Akt/b-catenin pathway. Cancer Res (in press).
Coleman, R., Gnant, M., Morgan, G., and Clézardin, P. (2012) Effects of bone-targeted agents on cancer progression and mortality. J Natl Cancer Inst 104, 1059-1067.
Dickinson, R.E., and Duncan, W.C. (2010) The SLIT-ROBO pathway: a regulator of cell function with implications for the reproductive system. Reproduction 139, 697-704.
Fradet, A., Sorel, H., Bouazza, L., Goehrig, D., Dépalle, B., Bellahcène, A., Castronovo, V., Follet, H., Descotes, F., Aubin, J.E., et al. (2011) Dual function of ERRα in breast cancer and bone metastasis formation: implication of VEGF and osteoprotegerin. Cancer Res 71, 5728-5738.
Garcia, T., Jackson, A., Bachelier, R., Clément-Lacroix, P., Baron, R., Clézardin, P., and Pujuguet, P. (2008). A convenient clinically relevant model of human breast cancer bone metastasis. Clin Exp Metastasis 25,33-42.
Jones CA, Nishiya N, London NR, Zhu W, Sorensen LK, Chan AC, Lim CJ, Chen H, Zhang Q, Schultz PG, et al. (2009) Slit2-Robo4 signalling promotes vascular stability by blocking Arf6 activity. Nat Cell Biol 11, 1325-1331.
Marlow, R., Strickland P, Lee JS, Wu X, Pebenito M, Binnewies M, Le EK, Moran A, Macias H, Cardiff RD, et al. (2008) SLITs suppress tumor growth in vivo by silencing Sdfl/Cxcr4 within breast epithelium. Cancer Res 68, 7819-7827.
Mehlen, P., Delloye-Bourgeois, C., and Chédotal, A. (2011). Novel roles for Slits and netrins: axon guidance cues as anticancer targets? Nat Rev Cancer 11, 188-197.
Nguyen, D.X., Bos, P.D., and Massagué, J. (2009). Metastasis: from dissemination to organ-specific colonization. Nat Rev Cancer 9, 274-284.
Peyruchaud, O., Serre, C. M., NicAmhlaoibh, R., Fournier, P., and Clézardin, P. (2003). Angiostatin inhibits bone metastasis formation in nude mice through a direct anti-osteoclastic activity. J Biol Chem 278, 45826-45832.
Prasad, A., Paruchuri, V., Preet, A., Latif, F., Ganju, R.K. (2008) Slit-2 induces a tumor-suppressive effect by regulating beta-catenin in breast cancer cells. J Biol Chem 283, 26624-26633.
Sin, S., Bonin, F., Petit, V., Meseure, D., Lallemand, F., Bièche, I., Bellahcène, A., Castronovo, V., de Wever, O., Gespach, C., et al. (2011) Role of the focal adhesion protein kindlin-1 in breast cancer growth and lung metastasis. J Natl Cancer Inst 103, 1323-1337.
Sun, H., Dai, K., Tang, T. and Zhang, X. (2009) Regulation of osteoblast differentiation by Slit2 in osteoblastic cells. Cells Tissues Organs 190, 69-80.
Weilbaecher, K.N., Guise, T.A., and McCauley, L.K. (2011) Cancer to bone: a fatal attraction. Nat Rev Cancer 11, 411-424.
Ypsilanti, A.R., Zagar, Y., Chédotal, A. (2010) Moving away from the midline: new developments for Slit and Robo. Development 137, 1939-1952.
Yu, J., Cao, Q., Yu, J., Wu, L., Dallol, A., Li, J., Chen, G., Grasso, C., Cao, X., Lonigro, R.J., et al. (2010) The neuronal repellent SLIT2 is a target for repression by EZH2 in prostate cancer. Oncogene 29, 5370-5380.

## Claims

1. An agent selected from the group consisting of an anti-ROBO4 antibody, a ROBO4 decoy polypeptide, and an inhibitor of ROBO4 expression for use in a method for preventing or treating bone metastases in a subject in need thereof wherein the inhibitor of ROBO4 expression is selected from the group consisting of ROBO4 siRNAs, ROBO4 shRNAs, ROBO4 ribozymes and ROBO4 antisense oligonucleotides and wherein the ROBO4 decoy polypeptide comprises the extracellular domain of ROB04.

2. The agent for use according to claim 1 wherein the subject suffers from a cancer selected from the group consisting of prostate cancer, breast cancer, lung cancer, melanoma, pancreatic cancer, colorectal cancer, ovarian cancer and brain cancer.

3. The agent for use according to claim 1 wherein the antibody is selected from the group consisting of chimeric antibodies, humanized antibodies and full human monoclonal antibodies.

4. The agent for use according to claim 1 wherein the ROBO4 decoy polypeptide comprises the extracellular domain of ROBO4 fused to the Fc domain of an immunoglobulin.

5. The agent for use according to claim 1 wherein the agent is administered to the subject in combination with bisphosphonates

6. The agent for use according to claim 1 wherein the agent is administered to the subject in combination with an anti-RANKL antibody.

7. The agent for use according to claim 6 wherein the anti-RANKL antibody is denosumab.

8. A method of testing whether a patient with a cancer is at risk of having bone metastases comprising i) determining the expression level of ROBO4 in a sample obtained from said patient ii) comparing the level determined at step i) with a predetermined reference level and iii) concluding that the patient has a high risk of having bone metastases when the level determined at step i) is higher than the predetermined reference level.

9. The method of claim 8 wherein the sample is isolated from the primary tumor of the patient or consists of a collection of circulating tumor cells isolated from the blood sample obtained from the subject.

10. The method of claim 8 wherein the subject suffers from a cancer selected from the group consisting of prostate cancer, breast cancer, lung cancer, melanoma, pancreatic cancer, colorectal cancer, ovarian cancer and brain cancer.

11. The method of claim 8 wherein the expression level of ROB04 in the sample is assessed by the determining the number of cancer cells expressing ROBO4 or the level of ROBO4 at the surface of the cancer cells.

## Patentansprüche

1. Mittel, ausgewählt aus der Gruppe bestehend aus einem Anti-ROBO4-Antikörper, einem ROBO4-Köderpolypeptid, und einem Inhibitor der ROBO4-Expression zur Anwendung in einem Verfahren zur Prävention oder Behandlung von Knochenmetastasen bei einem Patienten, der diese benötigt, wobei der Inhibitor der ROBO4-Expression ausgewählt ist aus der Gruppe bestehend aus ROBO4 siRNAs, ROBO4 shRNAs, ROBO4-Ribozymen und ROBO4 Antisense-Oligonukleotiden und wobei das ROBO4-Köderpolypeptid die extrazelluläre Domäne von ROBO4 umfasst.

2. Mittel zur Anwendung nach Anspruch 1, wobei der Patient an einem Krebs leidet, der ausgewählt ist aus der Gruppe bestehend aus Prostatakrebs, Brustkrebs, Lungenkrebs, Melanom, Bauchspeicheldrüsenkrebs, Darmkrebs, Eierstockkrebs und Gehirnkrebs.

3. Mittel zur Anwendung nach Anspruch 1, wobei der Antikörper ausgewählt ist aus der Gruppe bestehend aus chimären Antikörpern, humanisierten Antikörpern und vollständig humanen monoklonalen Antikörpern.

4. Mittel zur Anwendung nach Anspruch 1, wobei das ROBO4-Köderpolypeptid die extrazelluläre Domäne von ROBO4 umfasst, die an die Fc Domäne eines Immunglobulins fusioniert ist.

5. Mittel zur Anwendung nach Anspruch 1, wobei das Mittel dem Patienten in Kombination mit Bisphosphonaten verabreicht wird.

6. Mittel zur Anwendung nach Anspruch 1, wobei das Mittel dem Patienten in Kombination mit einem Anti-RANKL-Antikörper verabreicht wird.

7. Mittel zur Anwendung nach Anspruch 6, wobei der Anti-RANKL-Antikörper Denosumab ist.

8. Verfahren zum Testen, ob ein Patient mit Krebs dem Risiko ausgesetzt ist, Knochenmetastasen zu haben, umfassend i) Bestimmen des Expressionsniveaus von ROBO4 in einer Probe, die von dem Patienten erhalten wurde, ii) Vergleichen des in Schritt i) bestimmten Niveaus mit einem vorbestimmten Referenzniveau und iii) Schlussfolgern, dass der Patient einem hohen Risiko ausgesetzt ist, Knochenmetastasen zu haben, wenn das in Schritt i) bestimmte Niveau höher als das vorbestimmte Referenzniveau ist.

9. Verfahren nach Anspruch 8, wobei die Probe aus dem Primärtumor des Patienten isoliert wird oder aus einer Sammlung von zirkulierenden Tumorzellen besteht, die aus der von dem Patienten erhaltenen Blutprobe isoliert werden.

10. Verfahren nach Anspruch 8, wobei der Patient an einem Krebs leidet, der aus der Gruppe ausgewählt ist bestehend aus Prostatakrebs, Brustkrebs, Lungenkrebs, Melanom, Bauchspeicheldrüsenkrebs, Darmkrebs, Eierstockkrebs und Gehirnkrebs.

11. Verfahren nach Anspruch 8, wobei das Expressionsniveau von ROBO4 in der Probe durch das Bestimmen der Anzahl der Krebszellen, die ROBO4 exprimieren, oder des Niveaus von ROBO4 an der Oberfläche der Krebszellen bewertet wird.

## Revendications

1. Agent sélectionné dans le groupe constitué d'un anticorps anti-ROBO4, d'un polypeptide leurre de ROBO4, et d'un inhibiteur de l'expression de ROBO4 pour son utilisation dans un procédé de prévention ou de traitement des métastases osseuses chez un sujet le nécessitant dans lequel l'inhibiteur de l'expression de ROBO4 est sélectionné dans le groupe constitué d'ARNsi de ROBO4, d'ARNsh de ROBO4, de ribozymes de ROBO4 et d'oligonucléotides antisens de ROBO4 et dans lequel le polypeptide leurre de ROBO4 comprend le domaine extracellulaire de ROBO4.

2. Agent pour son utilisation selon la revendication 1 dans lequel le sujet souffre d'un cancer sélectionné dans le groupe constitué du cancer de la prostate, du cancer du sein, du cancer du poumon, d'un mélanome, du cancer du pancréas, du cancer colorectal, du cancer de l'ovaire et du cancer du cerveau.

3. Agent pour son utilisation selon la revendication 1 dans lequel l'anticorps est sélectionné dans le groupe constitué d'anticorps chimériques, d'anticorps humanisés et d'anticorps monoclonaux entièrement humains.

4. Agent pour son utilisation selon la revendication 1 dans lequel le polypeptide leurre de ROBO4 comprend le domaine extracellulaire de ROBO4 fusionné au domaine Fc d'une immunoglobuline.

5. Agent pour son utilisation selon la revendication 1 dans lequel l'agent est administré au sujet en combinaison avec des bisphosphonates

6. Agent pour son utilisation selon la revendication 1 dans lequel l'agent est administré au sujet en combinaison avec un anticorps anti-RANKL.

7. Agent pour son utilisation selon la revendication 6 dans lequel l'anticorps anti-RANKL est le dénosumab.

8. Procédé de test du fait qu'un patient atteint d'un cancer risque de développer des métastases osseuses comprenant i) la détermination du niveau de ROBO4 dans un échantillon obtenu dudit patient ii) la comparaison du niveau déterminé à l'étape i) à un niveau de référence prédéterminé et iii) la conclusion que le patient présente un risque élevé de développer des métastases osseuses lorsque le niveau déterminé à l'étape i) est supérieur au niveau de référence prédéterminé.

9. Procédé selon la revendication 8 dans lequel l'échantillon est isolé de la tumeur primaire du patient ou est constitué d'une collecte de cellules tumorales circulantes isolées de l'échantillon de sang obtenu du sujet.

10. Procédé selon la revendication 8 dans lequel le sujet souffre d'un cancer sélectionné dans le groupe constitué du cancer de la prostate, du cancer du sein, du cancer du poumon, d'un mélanome, du cancer du pancréas, du cancer colorectal, du cancer de l'ovaire et du cancer du cerveau.

11. Procédé selon la revendication 8 dans lequel le niveau d'expression de ROBO4 dans l'échantillon est évalué par détermination du nombre de cellules cancéreuses exprimant ROBO4 ou du niveau de ROBO4 sur la surface des cellules cancéreuses.
